(19) Europäisches Patentamt
European Patent Office
Office européen des brevets

(11) Veröffentlichungsnummer: **0 058 335**
**B1**

# (12) EUROPÄISCHE PATENTSCHRIFT

(45) Veröffentlichungstag der Patentschrift: **26.06.85**

(21) Anmeldenummer: **82100699.6**

(22) Anmeldetag: **02.02.82**

(51) Int. Cl.⁴: **C 07 C 87/58**, C 07 C 85/11, C 08 G 18/32, C 08 G 18/30, C 08 G 18/12

## (54) Neue Diamine, ein Verfahren zu ihrer Herstellung, sowie ihre Verwendung als Kettenverlängerungsmittel bei der Herstellung von Polyurethankunststoffen.

(30) Priorität: **13.02.81 DE 3105362**

(43) Veröffentlichungstag der Anmeldung: **25.08.82 Patentblatt 82/34**

(45) Bekanntmachung des Hinweises auf die Patenterteilung: **26.06.85 Patentblatt 85/26**

(84) Benannte Vertragsstaaten: **BE CH DE FR GB IT LI**

(56) Entgegenhaltungen:
**US-A- 2 934 571**
**US-A- 2 986 576**
**US-A- 4 218 543**
**Patent Abstracts of Japan, Band 2, Nr. 67, 20. Mai 1978, Seite 632C78**
**Katalysatoren, Tenside und Mineralöladditive von J. Falbe und K. Hasserodt, Georg Thieme Verlag, Stuttgart 1978**

(73) Patentinhaber: **BAYER AG**
**Konzernverwaltung RP Patentabteilung**
**D-5090 Leverkusen 1 Bayerwerk (DE)**

(72) Erfinder: **Scholl, Hans-Joachim, Dr.**
**Rudolf-Sohm-Strasse 28**
**D-5000 Koeln 60 (DE)**

**EP 0 058 335 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue, als Homologen- und Isomerengemisch vorliegende, Alkylsubstituierte Phenylendiamine, ein Verfahren zu ihrer Herstellung, sowie ihre Verwendung als Kettenverlängerungsmittel bei der Herstellung von Polyurethankunststoffen.

Aromatische Diamine stellen eine bekannte Verbindungsklasse dar. Die einfachsten Vertreter dieser Verbindungsklasse, d. h. die isomeren Phenylen- und Toluylendiamine sind jedoch mit dem Nachteil behaftet, daß es sich um bei Raumtemperatur kristalline Substanzen handelt, und die daher als Reaktionspartner für organische Polyisocyanate, beispielsweise als Kettenverlängerungsmittel bei der Herstellung von Polyurethankunststoffen kaum Eingang in die Praxis gefunden haben.

In der US-PS 2 934 571 wird die Herstellung von langkettigen Dinitro-alkyl-benzolen beschrieben, die gemäß US-PS 2 986 576 zu den entsprechenden Diaminen weiterverarbeitet werden können. Bei den gemäß US-PS 2 934 571 als Ausgangsmaterial eingesetzten Alkylbenzolen handelt es sich, wie insbesondere den Ausführungsbeispielen zu entnehmen, um weitgehend definierte Verbindungen, die insbesondere eine verzweigte Oligopropylen-Seitenkette aufweisen. Zur Nitrierung dieser Kohlenwasserstoffe wird in der Vorveröffentlichung ein spezielles Nitrierverfahren vorgeschlagen, da klassiche Nitrierverfahren offensichtlich bei der Nitrierung der gemäß Vorveröffentlichung einzusetzenden Kohlenwasserstoffe zu den entsprechenden Dinitro-alkyl-benzolen versagen. Trotz des aufwendigen Verfahrens unter Mitverwendung von Schwefeltrioxidhaltiger Schwefelsäure entstehen beim Verfahren der US-PS 2 934 571 störende Restanteile an Mononitroverbindungen, wie eigene Untersuchungen zur Dinitrierung eines gemäß Beispiel V der Vorveröffentlichung hergestellten Dodecylbenzols belegen. Die Weiterverarbeitung dieser Zwischenstufe zu Diaminen führt zwangsläufig zu Diaminen mit einem unerwünschten Gehalt an Monoaminen, so daß die Diamine als Kettenverlängerer bei der Herstellung von Polyurethankunststoffen weitgehend unbrauchbar sind. Hierdurch wird auch verständlich, daß die Verfahrensprodukte der genannten Vorveröffentlichungen bislang keinen Eingang in die technische Polyurethanchemie finden konnten.

Es war die Aufgabe der vorliegenden Erfindung, einfache aromatische Diamine, d. h. solche, die nur einen aromatischen Kern und zwei aromatisch gebundene primäre Aminogruppen aufweisen, zur Verfügung zu stellen, welche nach einem einfachen Verfahren zugänglich und bei Raumtemperatur flüssig sind, die bei Raumtemperatur keine Kristallisationstendenz aufweisen, die ohne störende Anteile an Monoaminen sind, und die daher problemlos als Reaktionspartner für organische Polyiosocyanate bei der Herstellung von Polyisocyanat-Additionsprodukten einsetzbar sind. Diese Aufgabe konnte durch die Bereitstellung der nachstehend näher beschriebenen erfindungsgemäßen Verbindungen gelöst werden.

Gegenstand der Erfindung sind, als Homologen- und Isomerengemisch vorliegende Diamine der Formel

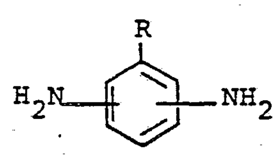

in welcher

R für einen gesättigten, geradkettigen, aliphatischen Kohlenwasserstoffrest mit 8-15 Kohlenstoffatomen steht,

und welche durch Dinitrierung und anschließende Hydrierung der Nitrogruppen von, als Homologen- und Isomerengemisch vorliegenden Kohlenwasserstoffen der Formel

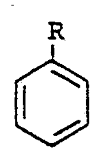

erhalten worden sind, die bei 1 013 mbar einen Siedebereich gemäß ASTM D 86 von 10-50 °C innerhalb des Temperabereichs von 270 °C-330 °C aufweisen.

Gegenstand der Erfindung ist auch ein verfahren zur Herstellung dieser neuen Diamine, welches dadurch gekennzeichnet ist, daß man als Homologen- und Isomerengemisch vorliegende Kohlenwasserstoffe der zuletzt genannten Art in an sich bekannter Weise durch Nitrierung in Dinitroverbindungen der Formel

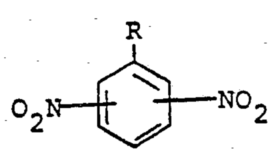

überführt und diese anschließend in an sich bekannter Weise zu den entsprechenden Diaminen hydriert.

Gegenstand der vorliegenden Erfindung ist auch die Verwendung der neuen Diamine als Kettenverlängerungsmittel bei der Herstellung von Polyurethankunststoffen nach dem Isocyanat-Polyadditionsverfahren.

Ausgangsmaterialien für das erfindungsgemäße Verfahren sind als Homologen- und Isomerengemisch vorliegende Alkylbenzolgemische der Formel

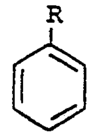

welche bei 1 013 mbar einen Siedebereich gemäß ASTM D 86 von 10-50 °C, vorzugsweise 20-30 °C innerhalb des Temperaturbereichs 270 °C-330 °C aufweisen, wobei vorzugsweise mindestens 30 Volumenprozent unterhalb der Mitte des jeweiligen Siedebereichs und mindestens 30 Volumenprozent oberhalb der Mitte des jeweiligen Siedebereichs sieden, und für welche

R für gesättigte, geradkettige aliphatische Kohlenwasserstoffreste mit 8-15, vorzugsweise 10-13 Kohlenstoffatomen steht.

Die Herstellung derartiger Alkylbenzol-Gemische erfolgt in an sich bekannter Weise durch Alkylierung von Benzol mit den entsprechenden technischen, linearen Olefingemischen, wie beispielsweise den entsprechenden technischen Oligoethylengemischen oder anderen linearen Oligoolefinen oder mit den entsprechenden technischen linearen Alkylchloridgemischen, wie beispielsweise in Kirk-Othmer, Encyclopedia of Chemical Technology, third edition, Vol. 2, (1978) auf Seiten 50 bis 61 beschrieben.

Bei der Durchführung des erfindungsgemäßen Verfahrens erfolgt die Überführung der Alkylbenzole in die entsprechenden Dinitroverbindungen durch eine an sich bekannte Nitrierungsreaktion beispielsweise im Temperaturbereich von 10 bis 30 °C unter Verwendung von beispielsweise 2-3 Mol konzentrierter Salpetersäure pro Mol Alkylbenzol, wobei die Salpetersäure in Form eines Gemischs mit konzentrierter Schwefelsäure (Nitriersäure) zur Anwendung gelangt. Bei den hierbei anfallenden Alkylsubstituierten Dinitrobenzolen sind gemäß Kernresonanzmessungen über 95 % aller Nitrogruppen in meta-Stellung zueinander angeordnet. Außerdem kann davon ausgegangen werden, daß ca. 10-30 Gew.-% der dinitrierten Alkylbenzole 1-Alkyl-2,6-dinitrobenzole und ca. 70-90 Gew.-% 1-Alkyl-2,4-dinitrobenzole darstellen. Diese Isomerenverteilung ist jedoch keineswegs erfindungswesentlich. So wäre es auch beispielsweise möglich, für die nächste Stufe des erfindungsgemäßen Verfahren dinitrierte Alkylbenzole einzusetzen, in denen andere Mengenverhältnisse der genannten Isomeren vorliegen. So könnte beispielsweise für die nächste Stufe des erfindungsgemäßen Verfahrens auch 1-Alkyl-2,4-dinitrobenzol in 2,6-freier Form eingesetzt werden, wie es beispielsweise durch zweistufige Nitrierung von Alkylbenzol über die Zwischenstufe des zunächst isolierten 1-Alkyl-4-nitrobenzols erhältlich ist.

Durch die Verwendung der erfindungswesentlichen Alkylbenzolgemische als Ausgangsmaterial werden die Dinitroalkylbenzolgemische ohne störende Mononitroanteile erhalten, wie der jeweils ermittelte Gehalt an Nitrogruppen, sowie dünnschichtchromatographische Untersuchungen der Nitrierungsprodukte belegen. Die so erhaltenen Dintroalkylbenzolgemische sind daher ausgezeichnet für eine direkte Weiterverarbeitung zu den erfindungsgemäßen Diaminen geeignet, ohne daß aufwendige Renigungsoperationen erforderlich werden, die insbesondere im Fall einer Entfernung der Mononitroverbindungen durch Destillation unter technischen Bedingungen unkalkulierbare Gefahren wegen der leichten Zersetzlichkeit von aromatischen Nitroverbindungen unter thermischer Belastung mit sich bringen würden.

Die nächste Stufe des erfindungsgemäßen Verfahrens besteht in der an sich bekanten Hydrierung der Nitrogruppen zu den entsprechenden primären Aminogruppen beispielsweise unter Verwendung von Raney-Nickel als Hydrierungskatalysator bei beispielsweise 20-60 °C und einem Wasserstoffdruck von beispielsweise 20 bis 40 bar.

Die bei dieser Hydrierung anfallenden erfindungsgemäßen Diamine der obengenannten Formel entsprechen selbstverständlich bezüglich der Stellung der Aminogruppen den bezüglich der Stellung der Nitrogruppen gemachten Ausführungen und bezüglich der Natur des Restes R den bei der Beschreibung der zu ihrer Herstellung einzusetzenden Alkylbenzolen gemachten Ausführungen.

Die erfindungsgemäßen Diamine stellen bei Raumtemperatur flüssige Substanzen dar, die keinerlei Kristallisationsneigung erkennen lassen, und die keinerlei störende Anteile an monofunktionellen Aminen enthalten. Es handelt sich bei den erfindungsgemäßen Verbindungen aus diesem Grund um wertvolle Kettenverlängerungsmittel bei der Herstellung von Polyurethankunststoffen nach dem Isocyanat-Polyadditionsverfahren.

Bei der erfindungsgemäßen Verwendung der neuen Diamine werden diese im allgemeinen mit NCO-Präpolymeren unter Einhaltung eines $NCO/NH_2$-Äquivalentferhältnisses von 0,7 : 1 bis 1,3 : 1, vorzugsweise 0,9 : 1 bis 1,1 : 1, in an sich bekannter Weise zur Reaktion gebracht. Bei den hierbei einzusetzenden NCO-Präpolymeren handelt es sich um an sich bekannte Umsetzungsprodukte der in der Polyurethanchemie an sich bekannten Diisocyanate mit unterschüssigen Mengen an Polyhydroxylverbindungen,

insbesondere Polyhydroxypolyestern oder Polyhydroxypolyethern eines zwischen 300 und 10 000, vorzugsweise 1 000 und 4 000 liegenden Molekulargewichts und einer (im Falle der Verwendung von Gemischen von Polyhydroxylverbindungen mittleren) Hydroxylfunktionalität von 2 bis 8, vorzugsweise 2 bis 3. Die neuen erfindungsgemäßen Diamine können jedoch auch vorteilhaft nach einem Einstufen-Verfahren bei der Herstellung von Polyurethanen verwendet werden, indem sie einer Polyhydroxylverbindung oder einem Gemisch von Polyhydroxylverbindungen der beispielhaft genannten Art zugemischt werden, worauf dieses Gemisch mit den bekannten Polyisocyanaten der Polyurethanchemie unter Einhaltung eines Äquivalentverhältnisses zwischen Isocyanatgruppen und gegenüber Isocyanatgruppen reaktionsfähigen Gruppen von ca. 0,8 : 1 bis 1,2 : 1, insbesondere 1 : 1 zum hochmolekularen Polyurethan umgesetzt wird. Dies ist im Falle der neuen erfindungsgemäßen Diamine insbesondere deswegen möglich, weil die ihnen fehlende Kristallisationstendenz auch auf die hierbei gebildeten Polyharnstoffe übertragen wird, so daß bei einem solchen Eintopf-Verfahren nicht mit unerwünschten Ausscheidungen von Polyharnstoffen gerechnet werden muß. Selbstverständlich kann dieses Verfahren beispielsweise unter Mitverwendung der in der Polyurethanchemie an sich bekannten Hilfs- und Zusatzmittel variiert werden, so daß beispielsweise bei Verwendung der an sich bekannten Treibmittel auch geschäumte Polyurethan-Polyharnstoffe zugänglich sind.

Die nachfolgenden Beispiele dienen der näheren Erläuterung der Erfindung. Alle Prozentangaben beziehen sich, falls nicht anderslautend erwähnt, auf Gewichtsprozente. Für die dünnschichtchromatograpischen Untersuchungen wurden DC-Fertigplatten, Kieselgel 60F-254, der Firma Merck AG verwendet.

Beispiel 1

In diesem Beispiel wird ein Homologengemisch linearer Alkylbenzole eingesetzt, deren Alkylketten eine Länge von 10 bis 13 C-Atomen bei einer mittleren Kettenlänge von etwa 12 C-Atomen aufweisen. Das Homologengemisch siedet gemäß ASTM D 86 bei 1 013 mbar zwischen ca. 283 und 313 °C, wobei ca. 50 volumenprozent bis 296 °C übergehen. Es handelt sich um ein Umsetzungsprodukt von Benzol mit einem Gemisch aus linearen $C_{10}$-$C_{13}$-Olefinen.

a) Nitrierung des Alkylbenzolgemischs :

In 1,97 kg Alkylbenzolgemisch wird eine Mischung von 1 136 ml 98 %iger Salpetersäure und 1 584 ml 96 %iger Schwefelsäure unter Kühlen so eingetropft, daß die Innentemperatur bei 10 bis 15 °C liegt. Nach Beendigung des Zutropfens wird 3 Stunden bei 25 bis 30 °C nachgerührt. Die organische Phase wird nachfolgend auf 10 kg Eis geschüttet, mit Natriumbicarbonatlösung neutral gewaschen und nochmals mit Wasser nachgewaschen. Die organische Phase wird abgetrennt und von verbleibenden Wasseranteilen durch Zentrifugieren weitgehend befreit. Das so erhaltene, flüssige Dinitroalkylbenzolgemisch wurde ohne weitere Reinigung der nächsten Reaktionsstufe zugeführt.

Ausbeute : 2,7 kg ; $NO_2$-Gehalt : 27,5 % (Theorie : 27,4 %). Nach dünnschichtchromatograpischen Untersuchungen waren keine Restanteile an Mononitroverbindungen nachzuweisen (Laufmittel 90 Gew.-Tle. Petrolether/10 Gew.-Tle. Ether).

b) Hydrierung des Dinitroalkylbenzolgemischs :

In einem Rührautoklaven werden 672 g der Dinitroverbindung gemäß a) in 1 700 ml Ethanol gelöst und mit 70 g Raney-Nickel versetzt. Bei 40 °C und 20 bis 40 bar Wasserstoffdruck wird bis zum Ende der Wasserstoffaufnahme gerührt. Anschließend wird entspannt, vom Katalysator abfiltriert und das Ethanol abdestilliert. Man erhält 550 g Rohamingemisch, welches als solches oder auch nach destillativer Reinigung für die erfindungsgemäße Verwendung geeignet ist. Das Rohamingemisch weist einen Stickstoffgehalt von 10,06 % (Theorie : 10,1 %) auf.

200 g Rohamingemisch werden unter vermindertem Druck destilliert. Auf diese Weise können 167 g Diamingemisch als bei 185 bis 204 °C/2,1 mbar siedende Fraktion erhalten werden (Ausbeute : 83,5 %).

Analyse (%) :

| | C | H | N |
|---|---|---|---|
| gefunden : | 78,6 | 11,5 | 10,0 |
| Theorie : | 78,3 | 11,6 | 10,1 |

(bezogen auf $C_{18}H_{32}N_2$)

Beispiel 2 (Vergleichsbeispiel)

a) Nitrierung von Dodecylbenzol :

Gemäß Beispiel V der US-PS 2 934 571 wurde Dinitrododecylbenzol hergestellt. Das Rohprodukt wies einen $NO_2$-Gehalt von nur 18,6 Gewichtsprozent auf. Nach Destillation gemäß Beispiel V der Vorveröffentlichung lag ein Produkt mit einem $NO_2$-Gehalt von 20,0 Gewichtprozent vor. Dünnschichtchromatographische Untersuchungen zeigen erhebliche Anteile an Mononitroverbindungen an, deren Vorliegen aus dem genannten niedrigen $NO_2$-Gehalt hergeleitet werden kann.

b) Hydrierung des Nitrierungsproduktes gemäß a) :

Gemäß US-PS 2 986 576, Beispiel V wurde die Nitroverbindung gemäß a) durch Hydrierung in die entsprechende Aminoverbindung überführt.

Analyse (%) :

| | C | H | N |
|---|---|---|---|
| gefunden : | 81,4 | 12,1 | 7,0 |
| Theorie : | 78,3 | 11,6 | 10,1 |

(bezogen auf $C_{18}H_{32}N_2$)

Beispiel 3 (Verwendungsbeispiel)

2 000 g eines linearen Polypropylenglykolethers (MG : 2 000, OH-Zahl : 56) werden mit 336 g 1,6-Diisocyanatohexan auf ca. 90 °C erwärmt und bis zu einem NCO-Gehalt von ca. 3,35 % bei dieser Temperatur gehalten.

500 g dieses NCO-Präpolymeren werden bei ca. 55 °C kurz entgast und innerhalb von 30 Sekunden mit 53 g des erfindungsgemäßen Diamingemisches aus Beispiel 1 vermischt. Nach eine Gießzeit von 4 bis 5 Minuten kann der Probekörper nach ca. 1,5 Stunden aus der auf 100 °C erwärmten Form entfernt werden. Nach 24-Stündigem Tempern bei 110 °C erhält man ein weiches Elastomeres (Shore A : 40) mit einer Elastizität von ca. 28 %.

Beispiel 4 (Vergleichsbeispiel zu Verwendungsbeispiel 3)

Entsprechend Beispiel 3 werden 500 g des dortgenannten NCO-Prepolymeren und 53 g des Amingemisches gemäß Beispiel 2 verarbeitet. Es entsteht lediglich eine Flüssigkeit der Viskosität 15 000 mPa.s/20 °C

**Patentansprüche**

1. Als Homologen- und Isomerengemisch vorliegende Diamine der Formel

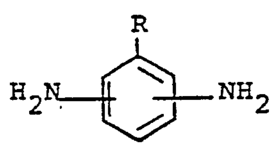

in welcher

R für einen gesättigten, geradkettigen, aliphatischen Kohlenwasserstoffrest mit 8-15 Kohlenstoffatomen steht

und welche durch Dinitrierung und anschließende Hydrierung der Nitrogruppen von, als Homologen- und Isomerengemisch vorliegenden Kohlenwasserstoffen der Formel

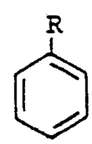

erhalten worden sind, die bei 1 013 mbar einen Siedebereich gemäß ASTM D 86 von 10-50 °C innerhalb des Temperaturbereichs von 270 °C-330 °C aufweisen.

2. Verfahren zur Herstellung von, als Homologen- und Isomerengemisch vorliegenden Diaminen gemäß Anspruch 1, dadurch gekennzeichnet, daß man die in Anspruch 1 genannten, als Homologen- und Isomerengemisch vorliegenden Kohlenwasserstoffe in an sich bekannter Weise durch Nitrierung in Dinitroverbindungen der Formel

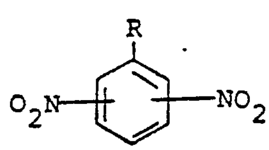

überführt und diese anschließend in an sich bekannter Weise zu den entsprechenden Diaminen hydriert.

3. Verwendung der als Homologen- und Isomerengemisch vorliegenden Diamine gemäß Anspruch 1 als Ketternverlängerungsmittel bei der Herstellung von Polyurethankunststoffen nach dem Isocyanat-Polyadditionsverfahren.

**Claims**

1. Diamines, present in the form of a mixture of homologues and isomers, of the formula

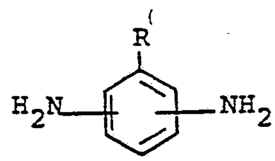

in which

R represents a saturated, straight-chain, aliphatic hydrocarbon radical with 8-15 carbon atoms, and which have been obtained by dinitration and subsequent hydrogenation of the nitro groups of hydrocarbons, present in the form of a mixture of homologues and isomers, of the formula

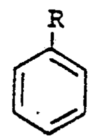

which have a boiling range according to ASTM D 86 of 10-50 °C at 1 013 mbar within the temperature range of 270 °C-330 °C.

2. Process for the preparation of diamines, present in the form of a mixture of homologues and isomers, according to Claim 1, characterised in that the hydrocarbons mentioned in Claim 1 and present in the form of a mixture of homologues and isomers, are converted in a manner known per se, by nitration, into dinitro compounds of the formula

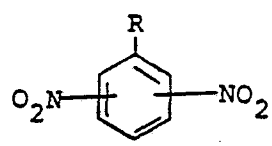

and these are then hydrogenated to give the corresponding diamines in a manner known per se.

3. Use of the diamines, present in the form of a mixture of homologues and isomers, according to Claim 1 as chain-lengthening agents in the production of polyurethane plastics by the isocyanate polyaddition process.

**Revendications**

1. Diamines présentes à l'état de mélanges d'homologues et d'isomères, de formule

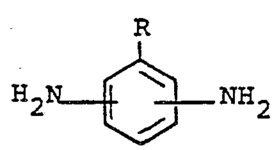

dans laquelle

R représente un reste hydrocarboné aliphatique saturé à chaîne droite en $C_8$-$C_{15}$,

et qui ont été obtenues par dinitration puis hydrogénation des groupes nitro d'hydrocarbures présents à l'état de mélanges d'homologues et d'isomères, de formule

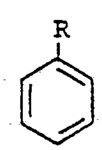

ayant, à 1 013 mbar, un intervalle d'ébullition selon ASTM D 86 de 10 à 50 °C à l'intérieur de l'intervalle de température de 270 à 300 °C.

2. Procédé de préparation des diamines présentes à l'état de mélanges d'homologues et d'isomères selon la revendication 1, caractérisé en ce que l'on convertit les hydrocarbures présents à l'état de mélanges d'homologues et d'isomères mentionnés dans la revendication 1, de manière connue en soi, par nitration, en composés dinitrés de formule

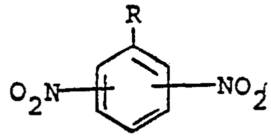

qu'on hydrogène ensuite de manière connue en soi en les diamines correspondantes.

3. Utilisation des diamines à l'état de mélanges d'homologues et d'isomères selon la revendication 1 en tant qu'agents d'allongement des chaînes à la préparation de résines synthétiques de polyuréthannes par le procédé de polyaddition des isocyanates.